Europäisches Patentamt

European Patent Office

Office européen des brevets

(11) Publication number: **0 279 669 B1**

(12) **EUROPEAN PATENT SPECIFICATION**

(45) Date of publication of patent specification: **27.12.95**

(51) Int. Cl.⁶: **C12N 15/02**, C12N 15/14, C12N 15/28

(21) Application number: **88301362.5**

(22) Date of filing: **18.02.88**

(54) **Cell surface marker of human pancreatic carcinoma cells**

(30) Priority: **19.02.87 US 16552**

(43) Date of publication of application:
**24.08.88 Bulletin 88/34**

(45) Publication of the grant of the patent:
**27.12.95 Bulletin 95/52**

(84) Designated Contracting States:
**BE CH DE FR GB IT LI NL SE**

(56) References cited:
**EP-A- 154 550**
**DE-A- 3 329 184**

**HYBRIDOMA, vol. 4, no. 1, 1985, page 77; P.J. MAIMONIS et al.: "The production of monoclonal antibodies reactive against a human pancreatic cancer cell line, COLO-357, and its variants"**

**CANCER RESEARCH, vol. 42, no. 2, February 1982, pages 601-608; R.S. METZGAR et al.: "Antigens of human pancreatic adenocarcinoma cells defined by murine monoclonal antibodies"**

(73) Proprietor: **SCRIPPS CLINIC AND RESEARCH FOUNDATION**
**10666 North Torrey Pines Road**
**La Jolla**
**California 92037 (US)**

(72) Inventor: **Kajiji, Shama**
**3915 Montefrio Court**
**San Diego**
**California 92130 (US)**
Inventor: **Quaranta, Vito**
**8861 Nottingham Place**
**La Jolla**
**California 92307 (US)**

(74) Representative: **Goldin, Douglas Michael et al**
**J.A. KEMP & CO.**
**14, South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 279 669 B1

BIOLOGICAL ABSTRACTS, vol. 80, no. 7, 1985, page AB-587, abstract no. 60241,Philadelphia, US; T. USUI et al.: "2 Novel monoclonal antibodies against human pancreatic cancer cell and their application", & SAPPORO MED. J., 54(2):155-166, 1985

CHEMICAL ABSTRACTS, vol. 102, no. 17, 29th April 1985, page 437, abstract no.147229q, Columbus, Ohio, US; W.H. SCHMIEGEL et al.: "Monoclonal antibody-defined human pancreatic cancer-associated antigens", & CANCER RES. 1985, 45(3), 1402-7

CANCER RESEARCH, vol. 47, no. 5, 1987, pages 1367-1376; S.M. KAJIJI et al.:"Six monoclonal antibodies to human pancreatic cancer antigens"

**Description**

BACKGROUND OF THE INVENTION

The present invention relates generally to novel hybridoma cell lines, and more specifically to monoclonal cell lines producing monoclonal antibodies reactive with human pancreatic cancer cells.

Cancer currently constitutes the second most common cause of death in the United States. Carcinomas of the pancreas are the eighth most prevalent form of cancer and fourth among the most common causes of cancer deaths in this country. The incidence of pancreatic cancer has been increasing steadily in the past twenty years in most industrialized countries, exhibiting the characteristics of a growing epidemiological problem.

The prognosis for pancreatic carcinoma is, at present, very poor, it displays the lowest five-year survival rate among all cancers. Such prognosis results primarily from delayed diagnosis, due in part to the fact that the early symptoms are shared with other more common abdominal ailments. The diagnosis of pancreatic cancer is often dependent on exploratory surgery, inevitably performed after the disease has advanced considerably.

Substantial efforts have been directed to developing tools useful for early diagnosis of pancreatic carcinomas. Nonetheless, a definitive diagnosis is often dependent on exploratory surgery which is inevitably performed after the disease has advanced past the point when early treatment may be effected. One promising method for early diagnosis of various forms of cancer is the identification of specific biochemical moieties, termed antigens present on the surface of cancerous cells. Antibodies which will specifically recognize and bind to the antigens present on the surfaces of cancer cells potentially provide powerful tools for the diagnosis and treatment of the particular malignancy. Tumor specific cell surface antigens have previously been identified for certain melanomas, lymphomas malignancies of the colon and reproductive tract.

There thus exists a great and long-felt need for a cell surface marker which is present on the surface of neoplastic cells of the pancreas, and for antibodies which specifically recognize such a cell surface marker. Such markers and corresponding antibodies would be useful not only in the early detection of pancreatic cancers, but in their treatment as well. The present invention satisfies these needs and provides related advantages as well.

SUMMARY OF THE INVENTION

The present invention provides a cell surface marker of human pancreatic carcinoma cells which is polyglycosylated peptide having

a MW of about 205 kd as determined by SDS-PAGE under reducing conditions and of about 190 kd under non-reducing conditions,

susceptibility to treatment with endoF to give a glycoprotein of 195 kd,

lack of susceptibility to endoH, and

a blocked N-terminus,

and the desialylated and other partially deglycosylated forms thereof, said peptide being capable of forming a heterodimer with an integrin α-chain of N-terminal sequence F-N-L-D-T-X-E-D-N-V-I-D-K-Y-G-D, wherein said heterodimer is specifically reactive with the monoclonal antibody S3-41 (produced by hybridoma ATCC MB 9318).

The invention further provides a cell surface marker comprising heterodimeric complex of MW 400 kd by SDS PAGE or 500 kd by gel filtration wherein said heterodimer has an α-chain of N-terminus F-N-L-D-T-X-E-D-N-V-I-D-K-Y-G-D, and a β-chain comprising a peptide as defined above.

The invention further provides monoclonal antibodies characterized in that the antibodies react specifically to the cell surface markers as defined above. These monoclonal antibodies, which recognize and bind to cell surface markers in HPC cells may be advantageously used for diagnosis and treatment of HPC.

In accordance with a further aspect of the invention, there are provided hybridoma cell lines which produce monoclonal antibodies specifically reactive with HPC cell surface markers. Preferred hybridoma cell lines are those termed S3-41 identified by ATCC accession numbers HB 9318, respectively, and the monoclonal antibodies produced by these cell lines.

It will be appreciated from the foregoing that the present invention provides novel markers for antibodies against HPC tumor cells. In one aspect of the invention, the monoclonal antibodies are used for in vitro immunoassays to detect HPC. In another aspect of the invention, the monoclonal antibodies, conjugated with certain detectable labels, are useful as in vivo imaging agents for detecting HPC. Moreover, when conjugated with certain toxins, such monoclonal antibodies are useful for therapeutic treatment of HPC.

The cell surface markers of the invention are reactive with the antibodies of the invention. These markers are useful in characterization of the cells bearing them and in design of agonists and antagonists of their functions. The antigens reactive with S3-41 antibodies are members of the integrin super family.

Other features and advantages of the present invention will become apparent from the following detailed description which illustrates, by way of example, the principles of the invention.

BRIEF DESCRIPTION OF THE FIGURES

FIG. 1 shows two-dimensional gel analysis of immunoprecipitates obtained by reacting S3-41 with radiolabeled extracts of FG cells.

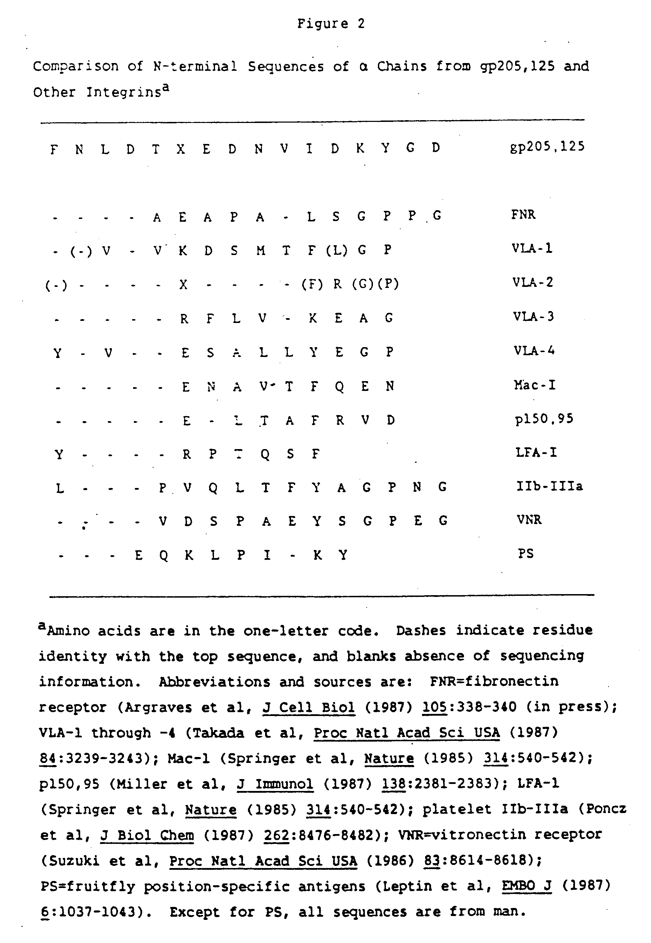

FIG. 2 shows a comparison of N-terminal sequences of various integrin $\alpha$-chains.

DETAILED DESCRIPTION OF THE PREFERRED EMBODIMENT

I. DEFINITIONS

"Monoclonal antibodies (Mabs) reactive with HPC" refers to homogenous populations of immunoglobulins which are capable of immunoreaction with antigens expressed on human pancreatic cancer (HPC) cells. It is understood that there may be a number of antigens present on the surface of any cell and, alternatively, that certain receptors present on HPC cells may also occur on other malignant or normal cell types. Moreover, such antigens may, in fact, have a number of antigenic determinants. The antibodies of the invention may be directed against one or more of these determinants. Any characteristic antigen associated with HPC may provide the requisite antigenic determinant.

Immunoglobulins, like all proteins, may exist in acidic, basic or neutral form depending on their amino acid composition and environment, and may be found in association with other molecules such as saccharides or lipids. The immunoglobulins of the present invention fall within the definition regardless of status in this regard as long as they remain capable of selectively reacting with HPC associated antigens.

"Cells" or "cell line" refers to the cells apparently denoted as well as the progeny thereof. It is known that during cell multiplication and growth cells or cell lines may not remain precisely constant in their genetic makeup and the progeny may, indeed, be distinguishable in some way from the parent cells. So long as the cells referred to herein retain the characteristic of secretion capability for Mabs reactive with HPC, as defined above, they are to be considered included in the definition.

"Immortalizing cell line" refers to a cell line which can be maintained perpetually, for practical purposes, in cell culture, i.e., for an indefinite number of transfers. It must also, when fused to an ordinary non-transformed cell line, which would normally not survive for more than a few days or weeks as a unicellular culture, be able to confer on the fusion product its own immortal properties.

A. GENERAL DESCRIPTION

The examples below describe the preparation of specific hybridoma cell lines producing monoclonal antibodies reactive with HPC cell antigens. It will be appreciated, however, that alternative methods may be employed to obtain alternative embodiments of the specific Mabs reactive with HPC cell antigens.

Techniques for preparing hybridomas are generally well-known in the art. Generally speaking, such hybridoma cell lines are prepared by a process involving the fusion under appropriate conditions of an immortalizing cell line and a B lymphocyte cell line appropriately immunized to produce the desired antibody. While the immortalizing cell lines so used are often of murine origin, those of any other mammalian species may be employed alternatively including those of rat, bovine, canine, human origin and the like. The immortalizing cell lines are most often of tumor origin, particularly myeloma cells, but may also include normal cells transformed with, for example, Epstein Barr Virus. Any immortalizing cell here may be used to prepare the hybridomas of the present invention.

Cells capable of secreting antibodies were employed as fusion partners, such as spleen cells or peripheral blood lymphocytes. The animal from which the cells were to be derived was immunized at intervals with whole cell suspensions of human pancreatic cancer cells. Alternatively, cell extracts or purified antigen may be used for immunization.

The immortalizing cells and lymphoid cells were fused to form hybridomas according to standard and well-known techniques employing polyethylene glycol as a fusing agent. Alternatively fusion may be accomplished by electrofusion. Hybridomas are screened for appropriate monoclonal antibody secretion by assaying the supernatant or protein purified from the ascites for reactivity with the desired cell or antigen. Such assay techniques include, among others, ELISA, RIA Western Blotting, or immunoprecipitation.

In the present invention, hybridomas were initially screened for production of antibodies reactive with HPC cells. Alternatively, HPC cell extracts or purified antigens could be used for screening. In order to further characterize the monoclonal antibodies, their reactivity with various HPC cell lines, other tumor cell lines and a variety of other normal, malignant and non-malignant pathological human tissues was determined using standard assay techniques such as ELISA, RIA, immunoprecipitation, histochemical staining procedures including indirect immunoperoxidase or indirect immunofluorescence staining. The hybridomas of the present invention were found to produce monoclonal antibodies generally highly reactive with all human pancreatic cell lines. They also displayed high reactivity with cells derived from other tumors, noticeably those of gastrointestinal and genitourinary tract origin. Moreover, although they displayed some reactivity with certain normal tissues, the Mab displayed negligible reactivity with major organs such as liver and kidney. Apparently, the antigens against which the antibodies are directed are highly expressed on HPC cells but only moderately or less on other cell types.

Because of their selective reactivity with HPC cell derived antigens the monoclonal antibodies are useful for both diagnosis and therapy of HPC and other carcinomas. Moreover, their non-reactivity with liver and kidney cells in particular permits them to be used therapeutically with relatively little risk of targeting these critical organs.

The antibodies of the invention are also useful in the preparation of cell surface markers reactive with them using immunoprecipitation and/or affinity chromatography of cell membrane preparations. The marker reactive with S3-41 antibodies described below is a member of the integrin super family, and is functional in mediating binding to the extracellular matrix. Accordingly, this marker is involved in metastasis and colonization of malignant cells.

## B. PARTICULAR EMBODIMENTS

The following examples illustrate a method for preparing hybridomas which can serve as sources for the desired monoclonal antibodies, and the antibodies thus produced. While the methods described are typical of those which might be advantageously used, other alternative procedures known to those skilled in the art may be alternatively employed. The examples are thus intended to illustrate, but not to limit the invention.

### EXAMPLE 1

### PREPARATION OF HYBRIDOMA CELL LINE

Murine Mabs reactive with HPC cell lines were produced essentially according to the standard techniques of Kohler and Milstein, Nature 256:495 (1975). Briefly, standard HPC cell lines such as COLO 357 and its subclones were used to obtain the antigenic preparation. Preferably cells of the cell line termed FG were employed. (Kajiji, S.M., Intraneoplastic Diversity in Human Pancreatic Cancer, Ph.D. Thesis, Brown University (1984). Alternatively other pancreatic cell lines expressing the antigens may be used, such as BxPC-3 (ATCC No. CRL1687). The cells were grown in a monolayer culture and harvested by EDTA treatment. Briefly, confluent monolayers were incubated for 20 minutes at 37°C with PBS containing 10 mM EDTA and 0.02% Kcl. The detached cells were collected, centrifuged at 1000 × g for 10 minutes and washed twice with cold PBS. Alternatively, cell suspensions were derived from FG xenografts grown in Balb/c athymic nude mice.

Two to four month old normal Balb/c mice were immunized with whole cell suspensions at weekly intervals with six intraperitoneal 0.5 ml injections, containing approximately $5 \times 10^6$ to $5 \times 10^8$ cells/injection/mouse. Three days after the final injection, the mice were sacrificed and the spleens removed. The spleens were placed in serum free Dulbecco's Minimal Essential Medium (DMEM) in separate Petri dishes and washed. The splenocytes were gently teased out of the fibrous splenic capsule using a rubber policeman. The cell suspension was then placed in a 15 ml tube and centrifuged at 1000 x g for 10 minutes. The pellet was then washed twice with serum-free DMEM.

The washed spleen cells and the P3X63Ag8 myeloma cells were fused according to the method of Kohler and Milstein, supra. The immortalized cell line fusion partners used were the murine myeloma cell line P3X63Ag8 (ATCC Accession No. T1B9) These myeloma cells were grown at a density of $5 \times 10^5$ cells/ml and harvested by centrifugation at 1000 x g for 10 minutes. The cell pellet was washed twice with serum free DMEM. Finally, the spleen cells and the P3X63Ag8 myeloma cells were combined at a ratio of 7:1 in a 50 ml tube and pelleted by centrifugation (1000 g for 10 minutes). The pellet was gently loosened and 1 ml of a 35% polyethylene glycol (PEG) solution was gently bubbled over the cells. After 1 minute, 1 ml of DMEM, containing 10% fetal calf serum (FCS)(Gibco, Grand Island, NY) was added to the cell suspension and gently mixed.

The PEG was subsequently diluted by the addition of 10 ml DMEM containing 10% FCS and the cells were repelleted. The cell pellet containing hybridoma fusion products was resuspended in 30 ml hypoxanthine-aminopterin-thymidine (HAT) medium (aminopterin from Sigma Chemical Co., St. Louis, MO; hypoxanthine and thymidine from Calbiochem, La Jolla, CA).

This cell suspension was then combined with 400 ml of HAT medium containing $2 \times 10^6$ thymocytes per ml (feeder cells). The contents were distributed into sterile 96 well plates (Costar, Cambridge, MA) and placed immediately in an incubator at 37°C. The spent media was replaced with fresh thymocyte containing HAT media after one week. Using this type of protocol successful hybridoma cultures were obtained which could be maintained with periodic addition of fresh DMEM containing 10% FCS.

Hybridomas producing monoclonal antibodies reactive with HPC cells were selected. After the cultures reached a cell density that covered 75-100% of the microtiter well surface, media from the hybridomas were screened for the presence of anti-HPC antibody, using a standard ELISA protocol. (Schultz, Cancer Res. 44:5914(1984)). Briefly, tumor cells dried onto the bottom of 96-well miniplates (Dynatech Microtiter Plates, American Scientific Products, McGaw Park, IL.) were used as targets. The wells of antigen-coated 96 well plates to be used were rinsed with buffer A pH 8.0 (20 mM Tris, containing 150 mM NaCl, 0.2% Tween 20 and 0.01% Thimerosal). The hybridoma supernatant diluted 1:2 in buffer B (buffer A containing 0.1% bovine serum albumin) was added to the wells and incubated for 1 hour at room temperature to permit binding of specific antibodies. Specifically bound antibodies were detected by adding horseradish peroxidase-conjugated rabbit anti-mouse immunoglobulin (BioRad, Richmond, CA) to wells that were rinsed free of the excess hybridoma supernatant by washing with buffer A. After incubation for 1 hour at room temperature the secondary antibody was decanted, the wells washed with buffer A, and 50 $\mu$l/well of substrate solution (ten milliliters of 80 mM citrate phosphate buffer, pH 5.0 containing 4 mg 0-phenylenediamine (Sigma Chem. Co., St. Louis, MO) and 4 $\mu$l 30% hydrogen peroxide) was added. The plates were incubated in the dark for 30 min at RT and the color reaction was stopped by adding 25 $\mu$l of 4 M sulfuric acid to each well. Specifically-bound antibodies were detected by measuring the absorbance at OD 490 on an ELISA scanner C model EL310, Biotek Instruments Winooski, VT) within 30 min. Reactivity was graded as follows: $A_{490} \leq 0.15$, -; $A_{490}$ = 0.15 to 0.3, 1+; $A_{490}$ = 0.3 to 0.6, 2+; $A_{490}$ = 0.6 to 1.2, 3+; $A_{490} \geq 1.2$, 4+. Hybridomas that were reactive with the immunizing FG cells but not with the lymphoblastoid 721-P cells were further screened for reactivity with frozen sections of HPC according to the procedure of Example II below. Only those that were reactive with frozen sections of HPC but not reactive with frozen sections of normal human liver, kidney and lung were selected. The hybridoma cell line selected for further study was designated S3-41.

EXAMPLE 2

CHARACTERIZATON OF MONOCLONAL REACTIVITY

A. REACTIVITY WITH HUMAN TUMOR TISSUES.

The reactivity of the monoclonal antibodies was determined by indirect immunoperoxidase staining as follows. Two- to 4-$\mu$m sections of frozen tissue blocks were cut on a cryotome, mounted on gelatin-coated glass slides, air-dried, and tested immediately in an indirect immunoperoxidase assay using the method of Taylor, Arch. Pathol. Lab. Med. 102:113 (1970). Briefly, after washing once in Hanks' balanced salt solution (Gibco, Grand Island, N.Y.) and phosphate buffered saline (PBS 10 mM sodium phosphate, 0.15 M Nacl, pH 7.0), sections were incubated at room temperature sequentially with: diluting buffer (PBS containing 5% normal goat serum and 1% bovine serum albumin) for 15 min; a 1:2 dilution of hybridoma supernatants or appropriate isotype-matched controls for one hour; horseradish peroxidase-conjugated goat anti-mouse Ig antiserum (Bio-Rad, Richmond, CA) diluted 1:50 and containing 5% normal human serum for one hour; and finally substrate buffer (10 mM Tris, pH 7.4, 0.6 mg/ml 3,3'-diaminobenzidine, 0.015% $H_2O_2$) for 15 min. Washes with HBSS and PBS were performed between incubations. Sections were counterstained in 1% methylene blue, dehydrated through graded ethanol, washed in Histo-Clear (National Diagnostics, Somerville, NJ), mounted in Pro-Texx (Lerner Laboratories, New Haven, CT), and examined by light microscopy.

Table 1 summarizes the reactivity of monoclonal antibodies produced by hybridoma cell lines S3-41 with 65 different tumors. As indicated, S3-41 was generally reactive only with carcinomas of the pancreas gastrointestinal tract, genitourinary tract, and head and neck tumors. Moreover, in virtually all instances, staining by the S3-41 Mabs was distinctly associated with the basement membranes surrounding tumor foci, producing a characteristic one-sided basal surface staining of cells at the epithelial stromal interface. In the few cases of lung carcinomas, melanoma and breast cancer tissues that were stained, reactivity was also confined to the basement membranes.

TABLE 1

REACTIVITY[a] OF MONOCLONAL ANTIBODIES WITH FRESH FROZEN HUMAN TUMOR TISSUE SECTIONS BY IMMUNOPEROXIDASE STAINING

|  | S3-41 |
| --- | --- |
| Pancreatic Cancer | |
| -ductal adenocarcinoma | -[b] |
| | 2+[b] |
| | 2+[b] |
| | - |
| | 4+[b] |
| | 3+[b] |
| | 4+ |
| -islet cell Cancer/insulinoma | - |
| | - |
| -acinar cell Cancer | - |
| Oral Squamous Cancer | 1[b] |
| | 1[b] |
| | - |
| | -[b] |
| Adenoid Cystic Cancer | 3+ |
| Salivary Gland Cancer | 4+[b] |
| Esophageal Cancer | 3+[b] |
| Gastric Cancer | 3+[b] |
| | 2+ |
| | 3+ |
| | 1+[b] |
| Colon Cancer | 3+[b] |
| | -[b] |
| | 3+ |
| | 3+[b] |

7

|  | S3-41 |
|---|---|
| Hepatoma | − |
|  | − |
| Laryngeal Cancer | $2+^b$ |
|  | $2+^b$ |
| Melanoma | $1+^b$ |
|  | − |
|  | − |
| Sarcoma | − |
|  | − |
| Lung Cancer |  |
|     -adenocarcinoma | $1+$ |
|  | − |
|  | − |
|     -squamous Cancer | $1+^b$ |
|  | − |
|  | − |
|  | − |
|  | − |
|     -adenosquamous | $1+^b$ |
|     -oat cell Cancer | − |
|  | − |
|     -large cell Cancer | $4+$ |
|     -mesothelioma | − |

|  | S3-41 |
| --- | --- |
| Breast Cancer | − |
|  | − |
|  | − |
|  | − |
|  | 1+ |
| Cervical Cancer | 2+[b] |
|  | 1+ |
| Endometrial Cancer | − |
|  | − |
|  | 3+[b] |
| Ovarian Cancer | − |
|  | − |
|  | 3+[b] |
| Prostatic Cancer | 1+[b] |
| Bladder Cancer | 1+[b] |
|  | 3+[b] |
| Kidney Cancer | − |
|  | 2+ |

## B. REACTIVITY WITH HUMAN CELL LINES

The reactivity of Mabs against a panel of cell lines in culture was determined by ELISA reactivity, according to the Method of Schultz, Cancer Res. 44:5914 (1984), as detailed in Example 1

Cells dried onto the bottom of 96-well miniplates were used as targets for ELISA. Horseradish peroxidase-conjugated goat anti-mouse Ig antiserum (Bio-Rad, Richmond, CA) was used as the secondary antibody.

The reactivity of Mab S3-41 is shown in Table 2. Mab S3-41 was reactive with the majority of the ten HPC cell lines tested. Moreover, it displayed particularly strong reactivity with cell lines derived from lung cancer, skin cancer and gastrointestinal and genitourinary tract tumors. Mab S3-41 was generally non-reactive with human red blood cells of blood types AB+, A+, B+, O+, and O-, normal diploid fibroblasts and leukemic or lymphoid cell lines.

9

## TABLE 2
## ELISA REACTIVITY OF MONOCLONAL ANTIBODIES
## WITH CULTURED HUMAN CELLS

| Cell lines (ATCC NO.) | S3-41 |
| --- | --- |
| Pancreatic Cancer | |
| Colo 357[a] | 3+ |
| FG[a] | 3+ |
| SG[a] | 3+ |
| FG-Met-2[a] | 4+ |
| RWP-1[a] | 4+ |
| RWP-2[a] | 3+ |
| PANC-1 (CRL 1469) | 3+ |
| ASPC-1 (CRL 1682) | 3+ |
| Hs 766T (HTB 134) | 4+ |
| BxPC-3 (CRL 1687) | 4+ |
| Lung Cancer | |
| -adenocarcinoma | |
| UCLA-P3[b] | - |
| A549 (CCL 185) | 2+ |
| CALU 6 (HTB 56) | - |
| -squamous cancer | |
| T-222[b] | 2+ |
| SK-MES-1 (HTB 58) | 3+ |
| CALU-1 (HTB 54) | 2+ |
| USCLS-1[b] | 3+ |
| -oat cell Cancer | |
| T-293[c] | - |
| NCI-H69 (HTB 119) | - |

| Cell lines (ATCC NO.) | S3-41 |
|---|---|
| Breast Cancer | |
| 734B[d] | − |
| BT-20 (HTB 19) | 3+ |
| MDA-MB-435S (HTB 129) | − |
| Bladder Cancer | |
| T24 (HTB 4) | − |
| J82 (HTB 1) | − |
| 5637 (HTB 9) | 2+ |
| Cervical Cancer | |
| ME-180 (HTB 33) | 3+ |
| Prostatic Cancer | |
| DU-145 (HTB 43) | 2+ |
| Pharyngeal Cancer | |
| FaDu (HTB 43) | 3+ |
| Skin cancer | |
| A-431 (CRL 1555) | 3+ |
| Colon Cancer | |
| COLO 396[d] | 4+ |
| Hepatoma | |
| SK-HEP-1 (HTB 52) | 3+ |
| Mesodermal Tumor | |
| SK-UT 1 (HTB 114) | − |

| Cell lines (ATCC NO.) | S3-41 |
| --- | --- |
| Melanoma | |
| ML-873-1[c] | - |
| WM239A[c] | - |
| WM2664 (CRL 1676) | - |
| A-375P[c] | - |
| A-375M[c] | - |
| M14[c] | - |
| M21[c] | - |
| MS-1[c] | - |
| FOSS[c] | - |
| Melur[c] | - |
| Glioblastoma | |
| U38MG (HTB 16) | - |
| U87MG (HTB 14) | - |
| U-373MG (HTB 17) | - |
| Neuroblastoma | |
| SK-N-SH (HTB 11) | - |
| SK-N-MC (HTB 10) | - |
| LAN-1[c] | 2+ |
| B-Lymphoblastoid | |
| L14[b] | - |
| LG-2[b] | - |
| 721-P[e] | - |
| GM3107[b] | - |

| Cell lines (ATCC NO.) | S3-41 |
|---|---|
| T-Lymphoblastoid | |
| MOLT-4 (CRL 1582) | - |
| HPB-ALL[b] | - |
| HSB-2[d] | - |
| Promyelocytic Leukemia | |
| HL-60 (CCL 240) | - |
| Erythroleukemia | |
| K562 (CCL 243) | - |
| Diploid Fibroblast | |
| W1-38 (CCL 75) | - |
| Human RBC | - |

Cell lines were obtained as follows:

a. P. Meitner, Department of Medicine, Brown University

b. L. Walker, Department of Immunology, Scripps Clinic and Research Foundation

c. R. Reisfeld, Department of Immunology, Scripps Clinic and Research Foundation

d. T. Edginton, Department of Immunology, Scripps Clinic and Research Foundation

e. F. Bach, University of Minnesota.

C. REACTIVITY WITH NON-MALIGNANT PATHOLOGIC HUMAN TISSUES

The reactivity of the Mab with a panel of inflammatory pancreases, benign tumor and hyperplastic epithelia was determined by indirect immunoperoxidase staining of frozen tissue sections, according to the method of section A, above. Mab S3-41 showed some reactivity with the duct cells of chronic pancreatitis tissues. Table 3 shows the results of testing with this panel of tissues.

TABLE 3

REACTIVITY OF MONOCLONAL ANTIBODIES WITH

FRESH FROZEN NON-MALIGNANT PATHOLOGIC HUMAN TISSUE

SECTIONS BY IMMUNOPEROXIDASE STAINING

| | S3-41 | | | |
|---|---|---|---|---|
| Pancreas (chronic pancreatitis) | | | | |
| acini | - | - | | |
| ducts | 2+ | -[b] | | |
| islets of Langerhans | - | - | | |
| Pancreas (SLE)[a] | | | | |
| acini | - | | | |
| ducts | -[b] | | | |
| islets of Langerhans | - | | | |
| Uterus (leiomyoma) | - | - | - | - |
| Ovary (fibroadenoma) | - | | | |
| Endometrium (hyperplastic) | 3+ | | | |
| Prostate (hyperplastic) | | | | |
| upper layers of epithelium | - | | | |
| basal layers of epithelium | 4+ | | | |
| basement membrane | 4+ | | | |

D. REACTIVITY WITH NORMAL ADULT AND FETAL TISSUES.

The reactivity of the Mabs with fresh frozen normal adult and fetal tissues was determined by indirect immunoperoxidase staining according to the method of section A, above. The antibodies were unreactive with the vast majority of normal tissues examined but displayed some reactivity with the basal epithelial layers or basement membranes of the esophagus, cervix, and large intestine, plantar skin, breast tissue and ileal epithelium. The restricted expression of the S3-41 antigen by the proliferating cell layers of normal stratified epithelia and its localization at the epithelial stromal interface suggests that this molecule may be an early differentiation antigen (possibly involved in cell adhesion) of epithelial cells that is re-expressed following malignant transformation. Further, the S3-41 antigen may be useful for diagnosis and therapeutic intervention of other skin-related disorders such as psoriasis and basal cell carcinomas and may prove to be a valuable cell surface marker for investigating epidermal cell biology.

Table 4 summarizes the results of this panel of tests.

TABLE 4

REACTIVITY OF MONOCLONAL ANTIBODIES

WITH FRESH FROZEN NORMAL HUMAN TISSUE

SECTIONS BY IMMUNOPEROXIDASE STAINING

|  | S3-41 |
|---|---|
| Esophagus | |
| stratified squamous epithelium | |
| -upper layers | - |
| -basal layers | 4+ |
| basement membrane | 4+ |
| Stomach | |
| gastric pits | - |
| gastric glands | |
| -parietal cells | - |
| -chief cells | - |
| lamina propria | - |
| Small Intestine | |
| jejunal epithelium | - |
| ileal epithelium | 3+ |
| basement membrane | 4+ |
| Large Intestine | |
| colonic epithelium | - |
| crypts of Lieberkuhn | - |
| basement membrane | 4+ |
| lamina propria | - |
| Liver | |
| parenchyma | - - - |
| bile ducts | - 1+ - |

15

|                       | S3-41   |
|-----------------------|---------|
| **Pancreas (adult)**  |         |
| acini                 | - - - - |
| ducts                 | - - -[a]- |
| islets of Langerhans  | - - - - |
| **Pancreas (fetal)**  |         |
| acini                 | -       |
| ducts                 | -[a]    |
| islets of Langerhans  | -       |
| **Thymus**            |         |
| cortex                | -       |
| medulla               | -       |
| **Lymph node**        |         |
| nodules               | -       |
| germinal centers      | -       |
| **Spleen**            |         |
| white pulp            | -       |
| red pulp              | -       |
| **Kidney (adult)**    |         |
| glomeruli             | - - -   |
| proximal tubules      | - - -   |
| distal tubules        | - - -   |
| **Kidney (fetal)**    |         |
| glomeruli             | -       |
| proximal tubules      | 1+      |
| distal tubules        | -       |

|                        | S3-41   |
|------------------------|---------|
| Cervix                 |         |
| columnar epithelium    | – 1+    |
| –basement membrane     | 4+ 4+   |
| squamous epithelium    |         |
| –upper layers          | – –     |
| –basal layers          | 4+ 4+   |
| –basement membrane     | 4+ 4+   |
| Uterus                 |         |
| endometrium            | 1+ –    |
| myometrium             | – –     |
| Ovary                  |         |
| cortex                 | –       |
| medulla                | –       |
| Breast                 |         |
| lobule                 | 4+ 2+   |
| duct                   | 4+ 3+   |
| basement membrane      | 4+ 4+   |
| Lung (adult)           |         |
| parenchyma             | – – –   |
| Lung (fetal)           |         |
| parenchyma             | –       |
| Thyroid                |         |
| epithelial cells       | –       |
| colloid                | –       |

17

|                      | S3-41 |
|----------------------|-------|
| Cerebrum             |       |
| cortex               | -     |
| Cerebellum           |       |
| granular layer       | -     |
| molecular layer      | -     |
| Purkinje cells       | -     |
| Plantar skin         |       |
| stratum corneum      | -     |
| stratum granulosum   | -     |
| stratum spinosum     | 1+    |
| stratum germinativum | 3+    |
| basement membrane    | 4+    |

[a]Basement membrane staining

E. REACTIVITY WITH CELL SURFACES

To determine whether the antigens recognized by the Mabs were expressed on surface of cells of reactive tissues, viable HPC cells were tested with the Mabs in indirect immunofluorescence assays as follows:

Cells grown to confluence on glass cover slips were washed once with cold HBSS, overlaid with 0.1 ml of 1:2 hybridoma supernatant for one hour at 4°C, washed in cold HBSS, and overlaid with 0.1 ml of 1:50 fluorescein isothiocyanate-conjugated goat anti-mouse Ig antiserum (Tago, Burlingame, CA) for one hour at 4°C. After washing and fixing in 3% paraformaldehyde, cells were mounted in 80% glycerol, 1 mg/ml p-phenylenediamine, 200 mM Tris, pH 8.5, examined and photographed with a Zeiss fluorescence microscope.

Mab S3-41 showed distinct staining of the plasma membrane, indicating recognition of cell surface structures. Both stained the entire cell population, displaying a contiguous, linear membrane pattern.

EXAMPLE 3

IMMUNOCHEMICAL CHARACTERIZATION OF ANTIGENS

In order to assess the chemical nature of the antigens recognized by Mabs, HPC cells were radiolabeled by incubation with either L-[³H]-leucine or [³H]-glucosamine, detergent solubilized and then subjected to immunoprecipitation with Mab immunosorbents, as follows:

Ten μl of a 10% suspension of protein-A-Sepharose (Pharmacia, Uppsala, Sweden) were incubated at 4°C for 1 hour with 5 μl of rabbit anti-mouse Ig antibodies (Accurate Chemicals, Westbury, NY) in 0.3 ml of PORT buffer (10 mM Tris, pH 8.5, 0.15 M NaCl, 0.5% Tween 20, 0.1% Renex 30, 2.5 mM sodium azide, 0.1% ovalbumin). After washing twice with PORT buffer, incubating 1 hour at 4°C with 1 ml hybridoma supernatants and washing twice with PORT buffer, the beads were incubated overnight at 4°C with radiolabeled cell extract ((1-2 x 107 cpm). The immunosorbents were washed 8 times with PORT buffer (10 mM Tris, pH 8.5, 0.15 M NaCl, 0.5% Tween 20, 0.1% Renex 30, 2.5 mM sodium azide) and bound antigens were eluted in Laemmli buffer (Nature 227:680(1970)). The samples were analyzed by SDS-PAGE on slab gels and visualized by fluorography.

18

Initial results of the SDS-PAGE analysis indicated that Mab S3-41 recognized a doublet protein antigen, of 205 kd and 135 kd, respectively. Both bands were glycosylated as they incorporated [$^3$H]-glucosamine. In some cases two additional bands of 150 kd and 185 kd were also seen. A band of 116 kd co-precipitated with S3-41 bands, but was non-specific since it could be removed by preabsorption with control immunosorbants.

Immunoprecipitation of a metabolically labeled HPC indicated that the antigenic determinants recognized by each of the monoclonals are carried by protein molecules. These proteins are also glycosylated, so that it remains to be determined whether the recognized epitopes are expressed by the protein or the glycan part of these molecules.

## EXAMPLE 4

## CHARACTERISTICS OF S3-41 ANTIGEN

Characterization of the antigen which immunoprecipitates with S3-41 demonstrates that it is a two-chain heterodimer which is a member of the integrin class of cell adhesion receptors. It contains two non-covalently bound glycopeptides, designated herein gp205 and gp125. The gp125 represents an analog to the $\alpha$-chains of other integrins, the gp205 peptide is analogous to other integrin $\beta$-chains, and has been shown to be polysialylated. This two-chain antigen has been shown to be expressed only on the basal lateral surfaces of epithelial cells and is evidently involved in adhesion.

Integrins in general are heterodimers wherein the $\alpha$-chains have high degrees of homology and the differences in the $\beta$-chain serve to place the various integrins into subfamilies. A review of the characteristics of integrins has been published by Hynes, R.O., Cell (1987) 48:459-554 and by Ruoslahti, E., et al, Science (1987) 238:491-497.

The integrin class of cell surface adhesion receptors is distinct from another type designated CAMs which are monomers which use polysialylation as a control element. The integrin to which S3-41 binds is distinguished by its polysialylation from other known integrins. The name intepsin has been proposed for this antigen.

## A. DISTRIBUTION OF THE ANTIGEN AND MOLECULAR WEIGHT OF $\alpha$- AND $\beta$-CHAINS

Immunohistology of human epidermal sections with S3-41 shows that staining is concentrated near the basement membrane and upper cell layers are progressively void of reactivity. Thus its expression is restricted to this particular portion of the cell surface.

FG-met2 pancreatic carcinoma cells were used as test substrates; lung carcinoma lines and short term normal human caratinocyte cultures gave identical results.

Cultures of FG-met2 pancreatic carcinoma cells were surface radioiodinated using $^{125}$-I sodium iodide in standard protocols. Detergent lysates of these labeled cells were immunoprecipitated with S3-41, and the immunoprecipitate applied to polyacrylamide gels under reducing and non-reducing conditions. Under reducing conditions, a 205 kd band was detected; under non-reducing conditions the single band appeared at 190 kd. This represents the $\beta$-chain of intepsin, designated herein gp205.

The $\alpha$-chain which migrates as a 150 kd band under non-reducing conditions and a 125 kd band under reducing conditions was detectable only when the cells were metabolically labeled either with [$^{35}$S]-methionine or with tritiated glucosamine. It is believed that the absence of the 125 kd/150 kd bands from the surface-labeled cells is due to an artifact of the iodination procedure.

That gp205 and gp125 components are non-covalently associated with each other at the cell surface was verified by treating FG-met2 cells (a human pancreatic carcinoma line) with a membrane impermeable cross-linker, DTSSP, and lysing the cells with detergent. This resulted in a 400 kd band upon SDS-PAGE. The 400 kd band disappeared and was replaced by 205 kd and 125 kd by subjecting the preparation to reduction, as DTSSP results in reversible cross-linking. The non-covalent complexing of gp205 and gp125 was further confirmed by immunoprecipitating protein from FG-met2 which had been labeled with [$^{35}$S]-methionine. The immunoprecipitate showed an approximate MW of 500 kd as analyzed by gel filtration. (The discrepancy in apparent MW is an artifact of the procedures.)

## B. PURIFICATION OF gp205 AND gp125

The surface proteins were isolated both from the lung adenocarcinoma cell line UCLA-P3 and from FG-met2.

The cells were grown in sufficient quantity to obtain 50 g wet weight and 20 g wet weight respectively. After washing, the cells were lysed in an equal volume of TBS containing 2% Renex 30, centrifuged at 10,000 × g for 30 min at 4°C and stored at -70°C.

The lysates were passed sequentially through a Sepharose column and through one or two sequential S3-41 immunosorbent columns at 5-10 ml/hr. After washing with TBS containing 0.1% Renex 30, pH 8.5 to remove unadsorbed material, the S3-41 column was inverted, washed with 3 column volumes of TBS, pH 8.5 containing 1.0% n-octyl β-D-glucopyranoside, and the bound material eluted with 50 mM diethylamine, pH 11.5 containing 150 mM NaCl, 1.0% n-octyl β-D-glucopyranoside. Eluted material was collected in 1.5 ml Eppendorf tubes containing 0.1 M Tris HCl pH 6.8, 150 mM NaCl, and 1.0% n-octyl β-D-glucopyranoside, to lower the pH rapidly to approximately 8.5. Elution was detected by SDS-PAGE stained with silver stain or Coomassie blue, and eluate from peaks containing protein was pooled and concentrated.

## C. CHARACTERIZATION OF PURIFIED ANTIGEN

Antigen corresponding to gp205 and to gp125, purified as above was subjected to amino acid sequencing from the N-termini using standard techniques. The gp125 fragment had the N-terminal sequence:

F-N-L-D-T-X-E-D-N-V-I-D-K-Y-G-D

which shows extensive homology with α N-terminal sequences of α-chains in other integrins, as illustrated in Figure 3.

The gp205 β-chain is apparently blocked at the N-terminus.

## D. METABOLIC LABELING WITH [35S]-METHIONINE IN THE PRESENCE OF TUNICAMYCIN

Indirect immunoprecipitation with Mab S3-41 of detergent lysates of cells intrinsically labeled with [35S]-methionine in the presence of tunicamycin (an inhibitor of N-linked glycosylation) and subsequent analysis by SDS-PAGE under reducing conditions revealed the presence of two major bands of 190 kd and 100 kd, respectively.

Semiconfluent cultures of FG cells were incubated for 10-12 hours at 37°C with 1 μg/ml tunicamycin. The cells were then pulsed for an additional 12 hours with 1 mCi [35S]-methionine in methionine-free RPMI medium containing 3% FCS and 1 μg/ml tunicamycin. Control flasks, similarly labeled with [35S]-methionine in the absence of tunicaymcin, were also prepared. The metabolically-labeled cells were then harvested as previously described and lysed using RIPA lysis buffer. Cell lysates were clarified by centrifugation at 100,000 x g for 45 min at 4°C and subsequent storage was at -70°C.

## E. TWO-DIMENSIONAL GEL ANALYSES

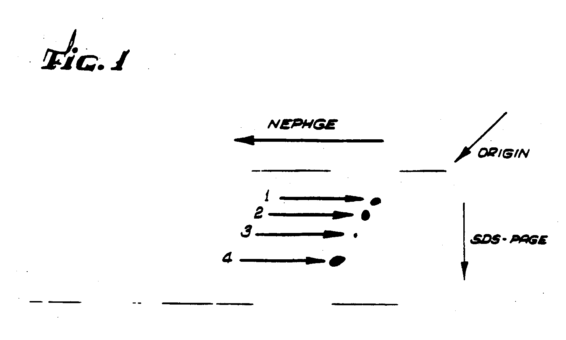

Immunoprecipitations were carried out by overnight incubation of cell lysates with immunoabsorbents prepared by activated-CNBr conjugation of Mab to Sepharose 4B-CL beads (Pharmacia, Uppsala, Sweden). After elution in 8 M urea at room temperature, samples were analyzed by two-dimensional electrophoresis, consisting of nonequilibrium pH electrophoresis on tube gels in the first dimension, followed by SDS-PAGE on 7.5 % acrylamide slab gels. Gels were impregnated with 2, 5,-diphenyloxazole, dried and exposed for the indicated times to Kodak XAR-5 X-ray film at -70°C. The resulting pattern of migration is shown in FIG. 1.

## F. CHARACTERIZATION OF THE gp205 AS A POLYSIALYLATE INTEGRIN β-CHAIN

Both gp205 and gp125, alone or together were treated with various enzymes which cleave carbohydrate chains.

EndoF and EndoH cleave N-linked complex oligosaccharides, and the results were consistent with one or two such oligosaccharides on gp205 and three or four on gp125:

EndoF:  205 kd → 195 kd
        125 kd → 105 kd
EndoH:  205 kd → 205 kd
        125 kd → 115 kd

Neuraminidase (NA) cleaves complex N-linked carbohydrates and polysialyl chains; endo-N-acetylneuraminidase (Endo-N) cleaves polysialylates only as α-2,8-linked linear sialic acid homopolymers. The results were consistent with gp205 as a polysialylated integrin β-chain:

NA:      205 kd → 95 kd

             125 kd → 120 kd

EndoN:   205 kd → 95 kd,150 kd

These results are consistent with a heavily polysialylated βintegrin chain wherein the sialylation is through β-linkage. (Most integrin β-chains have MW of 95-125 kd).

## G. BINDING TO LECTINS

When radiolabeled lysates were preabsorbed with lentil lectin-agarose beads (Vector Labs, Burlingame, CA), the antigens reactive with S3-41 were removed. Removal of antigen was shown by immunoprecipitation of bead supernatant, followed by SDS-PAGE. Similar treatment of radiolabeled lysates with wheat germ agglutinin-agarose beads (E.Y. Labs., San Mateo, CA) did not remove the S3-41 antigen. Therefore, the antigen reactive with S3-41 was characteristically bound to lentil lectin, but not to wheat germ agglutinin.

## H. GEL FILTRATION OF ANTIGEN

Radiolabeled detergent cell lysates were absorbed on wheat germ agglutinin-Sepharose columns (E.Y. Labs, San Meteo, CA), The breakthrough was absorbed onto lentil lectin-Sepharose columns. After washing, the absorbed material was eluted by the addition of 2% alpha-methyl-mannoside (Sigma Chemical Co., St. Louis). The eluted material was subjected to gel filtration, in the presence of 10 mM Tris, pH 8.0 containing 0.15 M NaCl, 1 mM CaCl$_2$, 1 mM MgCl$_2$.6H$_2$0, 0.02% sodium azide and 0.1% Renex 30 by using an FPLC instrument (Pharmacia, Uppsala, Sweden), equipped with a Sepharose 6 column. Molecular weight standards were run in parallel. One ml fractions were collected (approximately 40 fractions), and subjected to immunoprecipitation with S3-41 antibody. SDS-PAGE analysis of immunoprecipitates revealed that the following molecular species were reactive with S3-41 in the indicated fractions:

| SDS-PAGE m.w. | Fraction # | FPLC MW |
|---|---|---|
| 135; 205 | 25 | 669 kd |
| 135; 205 | 26 | 669 kd |
| 135; 150; 205 | 27 | approximately 669 kd |
| 135; 150; 205 | 28 | approximately 669 kd |
| 135; 150; 205 | 29 | between 669 kd and 440 kd |
| 135; 150 | 30 | between 669 kd and 440 kd |
| 135; only | 31-36 | between 440 kd and 232 kd |

The estimated molecular weights corresponding to the FPLC fractions are indicated in the right column. Since the estimated MW exceeds the MW determined by SDS-PAGE, the S3-41 antigens must exist as multimeric complexes, probably heterodimers formed by the association of one 205 kd component with one 125 kd component. The presence of excess free 125 kd component was also suggested by the material immunoprecipitated from fractions 31-36.

## I. PULSE CHASE BIOSYNTHETIC STUDIES:

Single cell suspensions of exponentially growing FG cells were propagated for one hour at 37°C in methionine-free medium (Irvine Scientific, Santa Ana, CA) and then pulse labeled for 10 min with [$^{35}$S]-methionine (1295 Ci/mM NEN Research Products, Boston, MA) at a concentration of 1.0-1.5 mCi/3×10$^7$ cells/ml. After the removal of an aliquot of 5×10$^6$ cells that constituted the zero-time point, the remaining cells were washed three times with cold Tris-buffer, pH 7.5 containing 10 mM unlabeled L-methionine (Sigma Chemical Co., St. Louis, MO). The labeled cells were resuspended in complete medium containing 10 mM unlabeled methionine and incubated on a shaker at 37°C. Aliquots were removed at the different time points indicated and the cells were centrifuged and extracted in RIPA lysis buffer as previously described.

After a 10 minute pulse with $[^{35}S]$-methionine, a faint band of 150 kd was detected at the zero time point of chase. This 150 kd component was clearly visible after 15 minutes of chase. Within the next 45 minutes of chase it appeared to be processed and the appearance of the 135 kd component was seen. Both the 205 kd and 135 kd molecules were detectable after 4 hours of chase until up to 20 hours of chase. It is presently not clear whether a precursor/product relationship exists between the two forms of the S3-41 antigen. While not wishing to be bound by the explanation, it appears that post-translational processing of the 150 kd molecule gives rise to the 135 kd subunit. Moreover, the 205 kd component could either arise by further processing of 135 kd component or by altered processing of the 150 kd precursor. Alternatively, it could have its own precursor molecule that is not recognized by Mab S3-41, thereby suggesting that the S3-41 antigen is a heterodimer comprised of two distinct non-covalently linked subunits.

EXAMPLE 5

THERAPEUTIC TREATMENT OF HPC

Patients determined to have HPC are treated with monoclonal antibodies reactive with HPC cells and conjugated with a toxin such as ricin, or any cytotoxic drug. The monoclonal antibody conjugates are administered (intravenously, intramuscularly, intraperitoneally, or the like, in a physiologically acceptable carrier solution, such as phosphate buffered saline. The dosage is determined by the body weight of the host, it preferably being in the range of about 0.1 mg/kg to about 40 mg/kg body weight, and usually about 1 mg/kg to about 10 mg/kg of host body weight. Alternatively, the dosage is established by evaluating the extent of the tumor as by quantitatively standardized ELISA, radioimaging or other methods. Treatment is repeated at intervals as necessary, to effect enhancement of the host's ability to recover from the infection.

EXAMPLE 6

IMAGING OF HPC TUMOR

Monoclonal antibodies reactive with HPC cells are utilized to determine the location and extent of HPC by methods well-known in the art, for example, Larson et al., Journal of Clinical Investigation 72:2101 (1983), which is incorporated by reference. Monoclonal antibodies are preferably radiolabeled by radioiodination or by other radiolabeling techniques well known in the art, such as chelation using a chelating agent such as diethylenetriaminepenta-acetic acid (DTPA); or are otherwise labeled, such as with agents having paramagnetic properties, with chemiluminescent substrates, or with components of an enzymatic reaction. The radiolabeled monoclonal antibodies are purified and formulated for pharmaceutical use. A solution of the labeled monoclonal antibodies in a carrier, for example in phosphate buffered saline, is injected intravenously into a host. The appropriate dose is in the range of about 100 $\mu$g to 50 mg. Time is permitted for the antibodies to migrate to regions of the body having concentrations of cells with antigenic determinants reactive therewith. Concentrations of radioisotypes in certain tissues are determined or may be mapped either by techniques of whole body imaging which are well-known in the art, (See, for example, Rainsbury et al., Lancet October 22, 1983, 934 (1983)) which is incorporated by reference, or by evaluating biopsied tissue or extracted body fluid using a scintillation counter. Where non-radioactive labels are used, other appropriate monitoring means are employed, such as a detector of nuclear magnetic resonance or a spectrophotometer. Areas of high radiation levels are indicative of the presence of cells such as HPC, having the cell surface markers of the present invention.

**Claims**

1.  A cell surface marker of human pancreatic carcinoma cells which is polyglycosylated peptide having
    a MW of about 205 kd as determined by SDS-PAGE under reducing conditions and of about 190 kd under non-reducing conditions,
    susceptibility to treatment with endoF to give a glycoprotein of 195 kd,
    lack of susceptibility to endoH, and
    a blocked N-terminus,
    and the desialylated and other partially deglycosylated forms thereof, said peptide being capable of forming a heterodimer with an integrin $\alpha$-chain of N-terminal sequence F-N-L-D-T-X-E-D-N-V-I-D-K-Y-G-D, wherein said heterodimer is specifically reactive with the monoclonal antibody S3-41 (produced by hybridoma ATCC MB 9318).

22

2. A cell surface marker comprising heterodimeric complex of MW 400 kd by SDS PAGE or 500 kd by gel filtration wherein said heterodimer has an α-chain of N-terminus F-N-L-D-T-X-E-D-N-V-I-D-K-Y-G-D, and a β-chain comprising a peptide as defined in claim 1.

3. A preparation of monoclonal antibody (Mab) specifically reactive with the cell surface marker of claim 1 or 2.

4. The Mab of claim 3 which is S3-41 (produced by ATCC HB 9318).

5. The hybridoma cell line ATCC HB 9318.

6. A monoclonal antibody as defined in claim 3 or 4 for use in a method for detecting or locating a pancreatic cancer in the body of a mammal, the method comprising the steps of:
   (a) administering to said mammal the monoclonal antibodies defined in claims 3 or 4 and
   (b) detecting or locating the administered Mab.

7. A monoclonal antibody as defined in claim 3 or 4 conjugated to a toxin.

8. A monoclonal antibody according to claim 7 for use in a method of therapeutically treating a patient having pancreatic cancer.

**Patentansprüche**

1. Zellenoberflächenmarkierer menschlicher Pankreaskarzinomzellen, der gebildet wird durch ein polyglycosyliertes Peptid mit
   einem Molekulargewicht von etwa 250 kd wie mittels SDS-PAGE unter reduzierenden Bedingungen bestimmt, und einem Molekulargewicht von 190 kd mittels SDS-PAGE unter nicht reduzierenden Bedingungen bestimmt,
   Empfänglichkeit gegenüber einer Behandlung mit endoF zur Schaffung eines Glycoproteins von 195 kd,
   keiner Empfindlichkeit gegenüber endoH, und
   einem blockierten N-Terminus,
   und desialylierte und andere teilweise deglycosylierte Formen davon, wobei das Peptid ein Heterodimer mit einer Integrin-α -Kette einer N-Terminussequenz F-N-L-D-T-X-E-D-N-V-I-D-K-Y-G-Dbilden kann, wobei das Heterodimer spezifisch reaktiv mit dem monoklonalen Antikörper S3-41 ist (hergestellt durch Hybridoma ATCC MB 9318).

2. Zellenoberflächenmarkierer mit einem Heterodimer-Komplex eines Molekulargewichts von 400 kd durch SDS-PAGE oder 500 kd durch Gelfiltration, wobei das Heterodimer eine α-Kette mit einem N-Terminus F-N-L-D-T-X-E-D-N-V-I-D-K-Y-G-D, sowie eine β-Kette mit einem wie in Anspruch 1 definierten Peptid aufweist.

3. Monoklonales Antikörperpräparat (Mab), welches spezifisch mit dem Zellenoberflächenmarkierer des Anspruchs 1 oder 2 reaktiv ist.

4. Monoklonaler Antikörper (Mab) nach Anspruch 3, welcher S3-41 (hergestellt durch ATCC HB 9318) ist.

5. Die Hybridoma-Zellinie ATCC HB 9318.

6. Monoklonaler Antikörper, wie in Anspruch 3 oder 4 definiert, zur Verwendung in einem Verfahren zur Feststellung oder Lokalisierung eines Pankreaskrebses im Körper eines Säugers, wobei das Verfahren die folgenden Schritte umfaßt:
   (a) Verabreichung der wie in Anspruch 3 oder 4 definierten monoklonalen Antikörper an den Säuger und
   (b) Feststellung oder Lokalisierung der verabreichten monoklonalen Antikörper (Mab).

7. Monoklonaler Antikörper, wie in Anspruch 3 oder 4 definiert, welcher zu einem Toxin konjugiert ist.

**8.** Monoklonaler Antikörper nach Anspruch 7 zur Verwendung in einem Verfahren zur therapeutischen Behandlung eines Patienten mit Pankreaskrebs.

**Revendications**

**1.** Marqueur de surface cellulaire de cellules de carcinome pancréatique humain qui est un peptide polyglycosylé ayant :
- une masse moléculaire d'environ 205 kd déterminées par SDS-PAGE dans des conditions réductrices et d'environ 190 kd dans des conditions non réductrices,
- une sensibilité à un traitement avec endoF pour donner une glycoprotéine de 195 kd,
- une absence de sensibilité à endoH, et
- une extrémité N-terminale bloquée,
- et les formes désialylées et autres formes partiellement déglycosylées de celui-ci, ledit peptide étant capable de former un hétérodimère avec une chaîne α d'intégrine de séquence N-terminale F-N-L-D-T-X-E-D-N-V-I-D-K-Y-G-D, où ledit hétérodimère est spécifiquement réactif avec l'anticorps monoclonal S3-41 (produit par hybridome ATCC MB 93 18).

**2.** Marqueur de surface cellulaire comprenant un complexe hétérodimère de masse moléculaire 400 kd par SDS PAGE ou 500 kd par filtration sur gel où ledit hétérodimère à une chaîne α d'extrémité N-terminale F-N-L-D-T-X-E-D-N-V-I-D-K-Y-G-D, et une chaîne β comprenant un peptide tel que défini dans la revendication 1.

**3.** Préparation d'anticorps monoclonal (Mab) spécifiquement réactif avec le marqueur de surface cellulaire de la revendication 1 ou 2.

**4.** Mab selon la revendication 3 qui est S3-41 (produit par ATCC HB 93 18).

**5.** Lignée cellulaire d'hybridome ATCC HB 93 18.

**6.** Anticorps monoclonal tel que défini dans la revendication 3 ou 4 pour utilisation dans une méthode pour la détection ou la localisation d'un cancer pancréatique dans le corps d'un mammifère, la méthode comprenant les étapes consistant à :
(a) administrer audit mammifère les anticorps monoclonaux définis dans les revendications 3 ou 4 et
(b) détecter ou localiser le Mab administré.

**7.** Anticorps monoclonal tel que défini dans la revendication 3 ou 4 conjugué à une toxine.

**8.** Anticorps monoclonal selon la revendication 7 pour utilisation dans une méthode de traitement thérapeutique d'un patient ayant un cancer pancréatique.

## Fig. 1

NEPHGE

ORIGIN

1

2

3

4

SDS - PAGE

Figure 2

Comparison of N-terminal Sequences of α Chains from gp205,125 and Other Integrins[a]

```
 F  N  L  D  T  X  E  D  N  V  I  D  K  Y  G  D        gp205,125


 -  -  -  -  -  A  E  A  P  A  -  L  S  G  P  P  G     FNR

 - (-) V  -  V  K  D  S  M  T  F (L) G  P              VLA-1

(-) -  -  -  -  X  -  -  -  -  - (F) R (G)(P)          VLA-2

 -  -  -  -  -  R  F  L  V  -  K  E  A  G              VLA-3

 Y  -  V  -  -  E  S  A  L  L  Y  E  G  P              VLA-4

 -  -  -  -  -  E  N  A  V- T  F  Q  E  N              Mac-I

 -  -  -  -  -  E  -  L  T  A  F  R  V  D              p150,95

 Y  -  -  -  -  R  P  T  Q  S  F                       LFA-I

 L  -  -  -  P  V  Q  L  T  F  Y  A  G  P  N  G        IIb-IIIa

 -  .  -  -  V  D  S  P  A  E  Y  S  G  P  E  G        VNR

 -  -  -  E  Q  K  L  P  I  -  K  Y                    PS
```

[a]Amino acids are in the one-letter code. Dashes indicate residue identity with the top sequence, and blanks absence of sequencing information. Abbreviations and sources are: FNR=fibronectin receptor (Argraves et al, J Cell Biol (1987) 105:338-340 (in press); VLA-1 through -4 (Takada et al, Proc Natl Acad Sci USA (1987) 84:3239-3243); Mac-1 (Springer et al, Nature (1985) 314:540-542); p150,95 (Miller et al, J Immunol (1987) 138:2381-2383); LFA-1 (Springer et al, Nature (1985) 314:540-542); platelet IIb-IIIa (Poncz et al, J Biol Chem (1987) 262:8476-8482); VNR=vitronectin receptor (Suzuki et al, Proc Natl Acad Sci USA (1986) 83:8614-8618); PS=fruitfly position-specific antigens (Leptin et al, EMBO J (1987) 6:1037-1043). Except for PS, all sequences are from man.